# EUROPEAN PATENT SPECIFICATION

(11) **EP 4 138 976 B1**
(45) Date of publication and mention of the grant of the patent: **17.09.2025**
(21) Application number: 20727073.7
(22) Date of filing: 30.04.2020
(51) Int. Cl.: A61M 25/09, A61M 25/06, A61M 29/00, A61M 25/00, A61M 25/01

(54) **CANNULATORS AND METHODS THEREOF**
KANÜLENBRENNER UND VERFAHREN DAFÜR
CANULATEURS ET PROCÉDÉS ASSOCIÉS

(43) Date of publication of application: 01.03.2023
(73) Proprietor: Bard Peripheral Vascular, Inc., Franklin Lakes, NJ 07417 (US)
(72) Inventor: ZIMMER, Brian, Salt Lake City, UT 84105 (US); FEDOR, Brenda, L. F., Holladay, UT 84117 (US); ISOM, Taylor, A., Sandy, UT 84070 (US); GLASS, Christopher, Scottsdale, AZ 85254 (US); HOLMES, Jonathan, Salt Lake, UT 84054 (US); JACKSON, Leslie, Tempe, AZ 85281 (US)
(74) Representative: Hoffmann Eitle
(86) International application number: PCT/US2020/030872
(87) International publication number: WO 2021/221679

(56) References cited:
- US-A1- 2004 171 988
- US-A1- 2012 041 371
- US-A1- 2012 197 200
- US-A1- 2015 224 287
- US-A1- 2020 094 025

## Description

### BACKGROUND

Existing dialysis needles are rigid (e.g., metal) with a sharp tip that is left in place during dialysis. The sharp tip of such dialysis needles can result in infiltration (e.g., puncture of a blood-vessel wall) when the sharp tip is incorrectly advanced or a patient moves during dialysis, the latter of which is likely due to the duration of a dialysis treatment procedure. A device is needed to reduce any potential for infiltration. Disclosed herein are cannulators that address the foregoing.
US 2020/0094025 A1 discloses a vasculature access device that includes a needle, a dilator, an introducer sheath, a hub, and manipulators in a single, integrated device. The vasculature access device also includes one or both of a guidewire and one or more features for facilitating restraint of the hub and the introducer sheath relative to a patient.US 2012/041371 A1, US 2012/197200 A1, US 2015/224287 A1, and US 2004/171988 A1 are further prior art.

### SUMMARY

The invention is defined by claim 1.

**In** some embodiments, the compound needle further includes an access guidewire disposed in the needle. The access guidewire extends from the cavity through a through hole in the distal wall of the needle hub.

**In** some embodiments, the compound needle further includes a guidewire actuator. The guidewire actuator is configured to advance a distal end of the access guidewire beyond the tip of the needle or withdraw the distal end of the access guidewire into a distal- end portion of the needle proximal of the tip of the needle.

**In** some embodiments, the guidewire actuator includes a slider slidably disposed in a longitudinal slot of the needle hub. The slider includes an extension coupled to a proximal end portion of the access guidewire in the cavity of the needle hub.

**In** some embodiments, the cannula is formed of a flexible polymeric material configured to increase patient comfort during a dialysis treatment procedure.

**In** some embodiments, a distal-end portion of the cannula includes a taper. The taper of the cannula is configured to continue from a taper of the dilator in the mated state of the cannulator.

In some embodiments, the cannula hub is furcated (e.g., bifurcated). When furcated, the cannula hub includes at least a primary arm and a secondary arm.

In some embodiments, the primary arm of the cannula hub includes a primary arm lumen and the secondary arm of the cannula hub includes a secondary-arm lumen. Each lumen of the primary-arm lumen and the secondary-arms lumen is fluidly connected to a cannula lumen of the cannula.

In some embodiments, each arm of the primary arm and the secondary arm of the cannula hub terminates in a Luer connector.

In some embodiments, the proximal-end portion of the cannula hub coupled to the distal-end portion of the needle hub in the mated state of the cannulator is the Luer connector of the primary arm of the cannula hub disposed in a bore in the distal-end portion of the needle hub.

Disclosed herein is a cannulator for cannulating an arteriovenous fistula. The cannulator includes, in some embodiments, a compound needle and a compound cannula. The compound needle includes an access guidewire, a needle, a dilator, and a needle hub. The needle is disposed over the access guidewire. The dilator is disposed over the needle with at least a tip of the needle extending beyond a distal end of the dilator. The needle hub includes a housing enclosing a cavity having a distal wall. Each of a proximal end of the needle and a proximal end of the dilator is coupled to the distal wall. The guidewire actuator is configured to advance a distal end of the access guidewire beyond the tip of the needle or withdraw the distal end of the access guidewire into a distal-end portion the needle proximal of the tip of the needle. The guidewire actuator includes a slider slidably disposed in a longitudinal slot of the needle hub. The slider includes an extension coupled to a proximal-end portion of the access guidewire that extends into the cavity of the needle hub through a through hole in the distal wall of the needle hub. The compound cannula includes a cannula and a cannula hub around a proximal-end portion of the cannula. The cannulator has a mated state of the compound needle and the compound cannula for at least cannulation with the cannulator. The mated state of the cannulator includes the dilator disposed in the cannula with a Luer connector in a proximal end portion of the cannula hub disposed in a bore of a distal-end portion of the needle hub.

In some embodiments, a distal-end portion of the cannula includes a taper. The taper of the cannula is configured to continue from a taper of the dilator in the mated state of the cannulator.

In some embodiments, the cannula hub is furcated (e.g., bifurcated). When furcated, the cannula hub includes at least a primary arm and a secondary arm.

In some embodiments, the primary arm of the cannula hub includes a primary arm lumen and the secondary arm of the cannula hub includes a secondary-arm lumen. Each lumen of the primary-arm lumen and the secondary-arms lumen is fluidly connected to a cannula lumen of the cannula.

Also disclosed herein but not part of the claimed subject-matter is a method of cannulating a patient with a cannulator. The method includes, in some embodiments, a cannulator-obtaining step, a puncture establishing step, an access guidewire-advancing step, a tissue-dilating step, a cannula advancing step, and a needle-withdrawing step. The cannulator-obtaining step includes obtaining the cannulator. The cannulator includes a compound needle and a compound cannula in a mated state of the cannulator. The puncture-establishing step includes establishing a percutaneous puncture by inserting a tip of a needle of the compound needle into a blood vessel of the patient. The access guidewire-advancing step includes advancing a distal end of an access guidewire beyond the tip of the needle and into a blood-vessel lumen with a guidewire actuator of the compound needle. The tissue-dilating step includes dilating tissue surrounding the puncture with a dilator of the compound needle disposed over the needle. The cannula advancing step includes advancing a cannula of the compound cannula over the dilator, the needle, and the access guidewire into the blood-vessel lumen. The needle-withdrawing step includes withdrawing the compound needle from the blood-vessel lumen leaving the compound cannula in place.

In some embodiments, the access guidewire-advancing step includes distally sliding a slider disposed in a longitudinal slot of the needle hub. The slider includes an extension coupled to a proximal-end portion of the access guidewire in a cavity of the needle hub.

In some embodiments, the method further includes an access guidewire withdrawing step. The access guidewire-withdrawing step includes withdrawing the distal end of the access guidewire into a distal-end portion of the needle proximal of the tip of the needle with the guidewire actuator before the needle-withdrawing step.

In some embodiments, the method further includes an ensuring step. The ensuring step includes ensuring the compound needle and the compound hub are properly mated with a Luer connector of a primary arm of the cannula hub disposed in a bore of a distal- end portion of the needle hub.

**In** some embodiments, the ensuring step includes ensuring a taper of a distal- end portion of the dilator continues along a taper of a distal-end portion of the cannula.

In some embodiments, the tissue-dilating step includes dilating the tissue with the distal-end portion of the cannula.

In some embodiments, the blood vessel is an arteriovenous fistula.

In some embodiments, the method further includes a dialysis machine connecting step and a dialysis-commencing step. The dialysis machine-connecting step includes connecting the compound cannula to a tubing set of a dialysis machine. The dialysis commencing step includes commencing dialysis through the compound cannula.

These and other features of the concepts provided herein will become more apparent to those of skill in the art in view of the accompanying drawings and following description, which describe particular embodiments of such concepts in greater detail.

### DRAWINGS

FIG. 1 illustrates a first isometric view of a cannulator in accordance with some embodiments.
FIG. 2 illustrates a second isometric view of the cannulator in accordance with some embodiments.
FIG. 3 illustrates a top view of the cannulator in accordance with some embodiments.
FIG. 4 illustrates a side view of the cannulator in accordance with some embodiments.
FIG. 5 illustrates an isometric view of a compound cannula separated from a compound needle of the cannulator in accordance with some embodiments.
FIG. 6 illustrates a side view of the compound cannula separated from the compound needle in accordance with some embodiments.
FIG. 7 illustrates an isometric cross-sectional view of a proximal-end portion of the compound needle in accordance with some embodiments.
FIG. 8 illustrates an isometric cross-sectional view of the compound cannula in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

As set forth above, existing dialysis needles are rigid (e.g., metal) with a sharp tip that is left in place during dialysis. The sharp tip of such dialysis needles can result in infiltration (e.g., puncture of a blood-vessel wall) when the sharp tip is incorrectly advanced or a patient moves during dialysis, the latter of which is likely due to the duration of a dialysis treatment procedure. A device is needed to reduce any potential for infiltration.

Disclosed herein are cannulators and methods thereof that address the foregoing.

### Cannulators

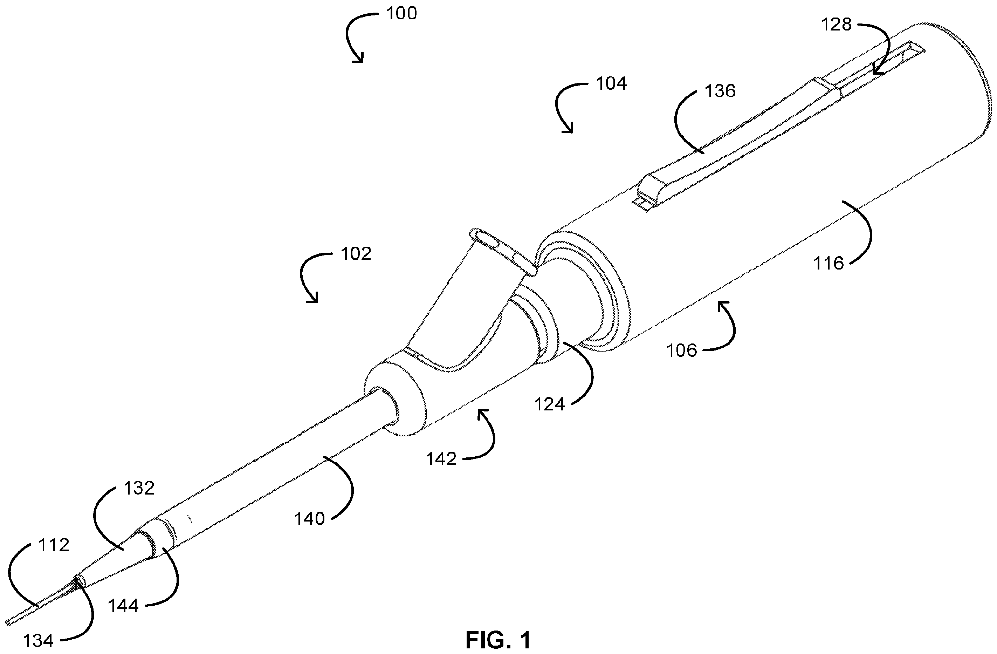

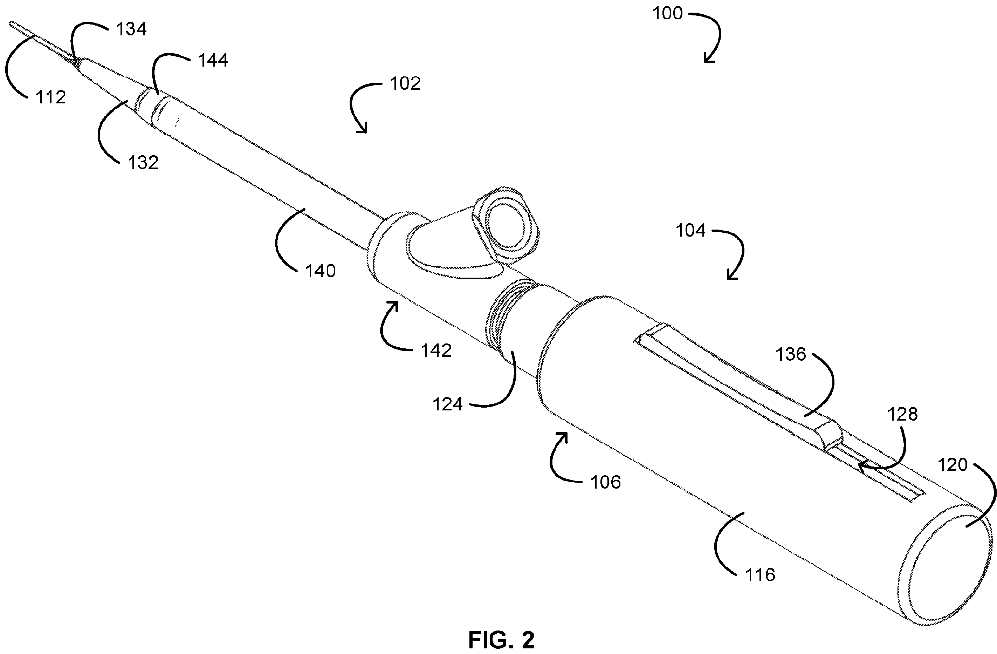

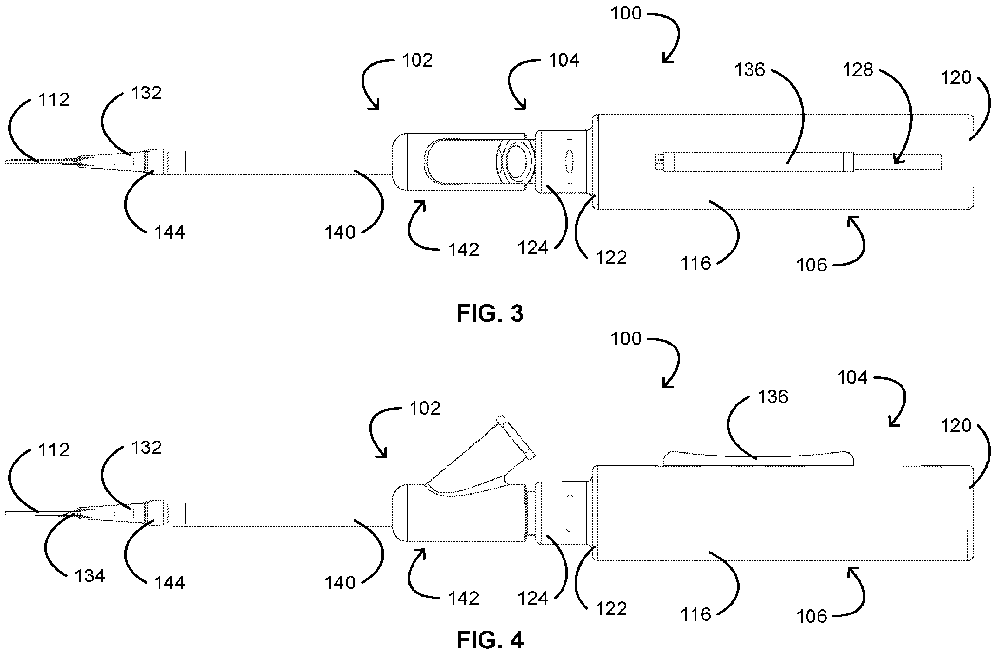

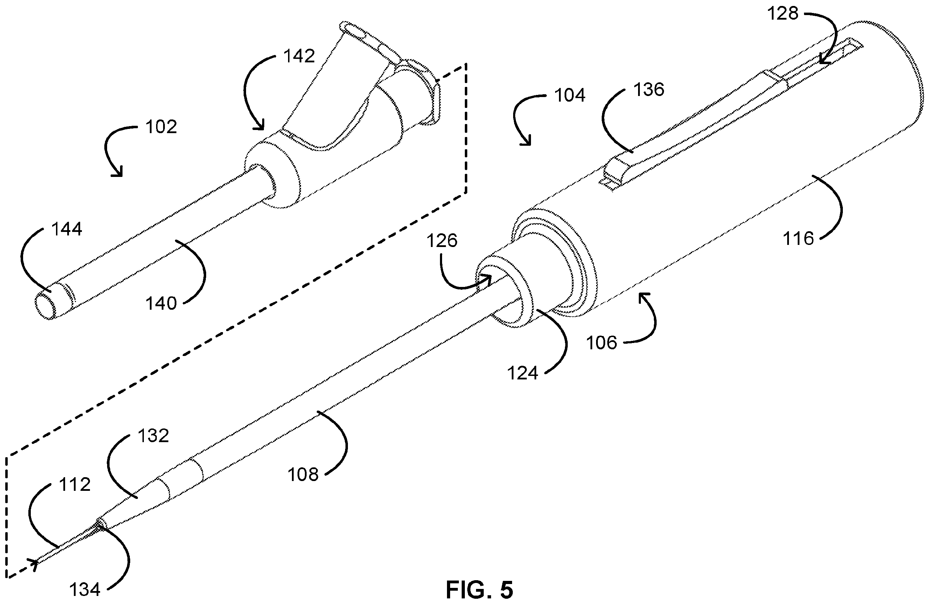

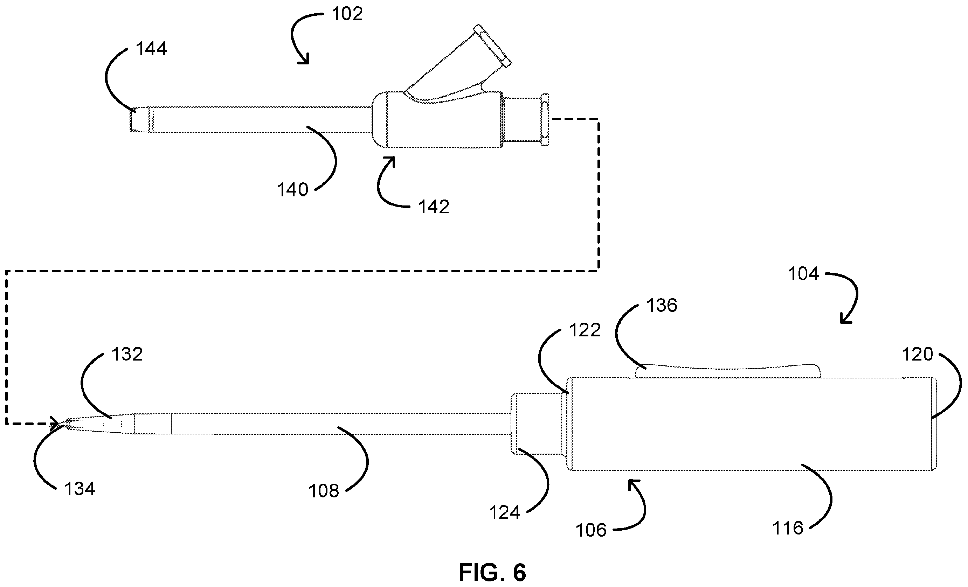

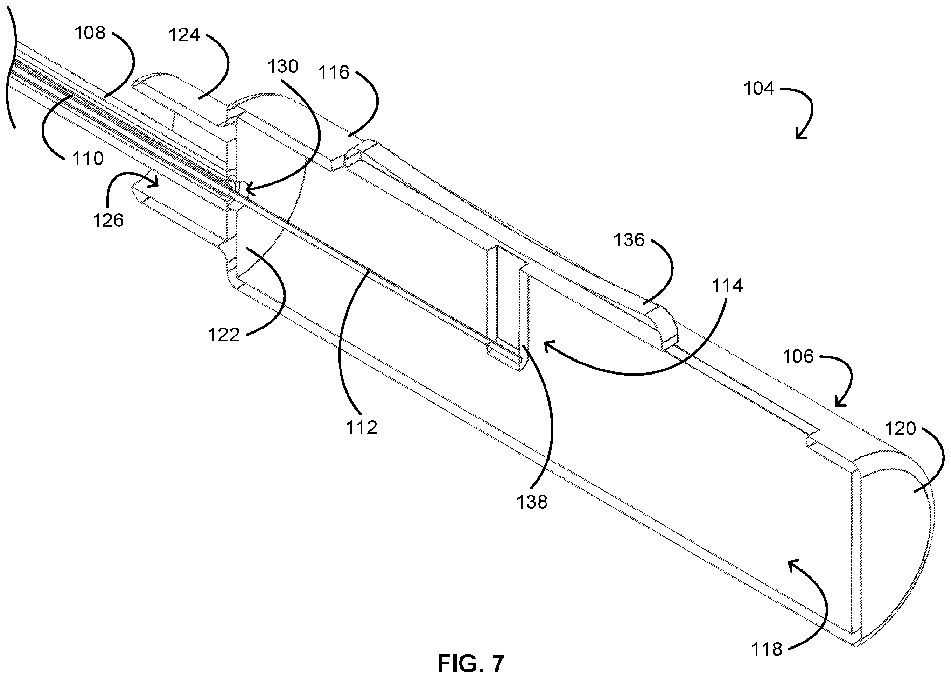

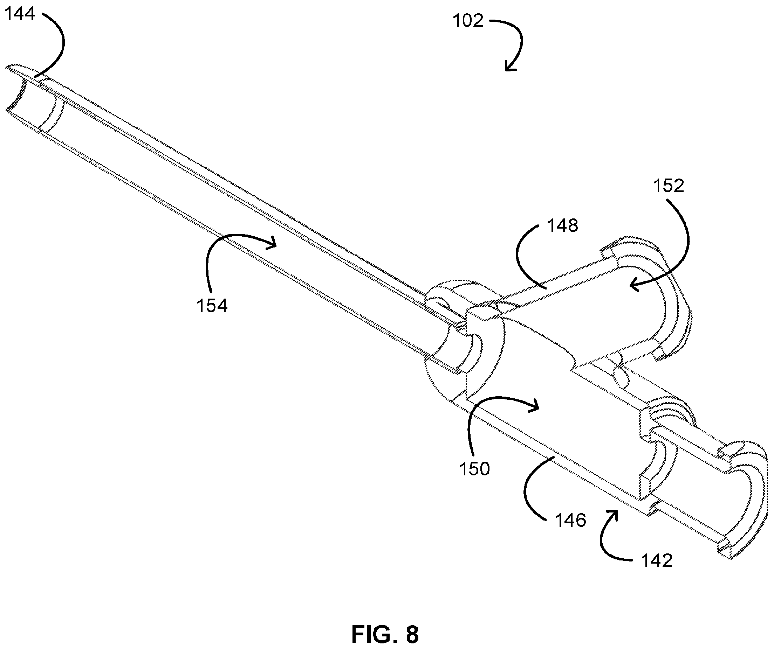

**FIGS.** 1-4 illustrate different views of a cannulator 100 in accordance with some embodiments. FIGS. 5 and 6 illustrate different views of a compound cannula 102 separated from a compound needle 104 of the cannulator 100 in accordance with some embodiments. FIG. 7 illustrates an isometric cross-sectional view of a proximal-end portion of the compound needle 104 in accordance with some embodiments. FIG. 8 illustrates an isometric cross-sectional view of the compound cannula 102 in accordance with some embodiments.

The cannulator 100 is configured for at least cannulating an arteriovenous fistula. As shown, the cannulator 100 includes the compound cannula 102 and the compound needle 104. The compound needle 104 and the compound cannula 102 are described, in turn, in sections set forth below; however, some crossover between the sections for the compound needle 104 and the compound cannula 102 exist in view of the interrelatedness of the compound needle 104 and the compound cannula 102 in the cannulator 100.

As shown, the compound needle 104 includes a needle hub 106, a dilator 108, a needle 110, and an access guidewire 112. The compound needle 104 can further include a guidewire actuator 114 for actuating (e.g., advancing or withdrawing) the access guidewire 112.

The needle hub 106 is configured to be held in a single hand as one might underhandedly hold a flashlight. Indeed, the needle hub 106 includes a housing 116 sized and shaped as a handle that can be cradled by fingers of a single hand while establishing a percutaneous puncture with the cannulator 100 as set forth below.

The housing 116 surrounds a cavity 118 and caps the cavity 118 with a proximal wall 120 and a distal wall 122, thereby forming a sterile enclosure for at least a proximal-end portion of the access guidewire 112. In addition, a distal-end portion of the needle hub 106 includes an extension 124 of the housing 116 including a bore 126 configured to mate with the connector of the primary arm 146 of the cannula hub 142 as set forth below.

The housing 116 can include a longitudinal slot 128 for the slider 136 of the guidewire actuator 114 when the guidewire actuator 114 is present in the compound needle 104. If the guidewire actuator 114 is not present, the access guidewire 112 can be advanced or withdrawn by way of through holes in the proximal and distal walls 120 and 122 such as the through hole 130 in the distal wall 122.

The dilator 108 includes a distal-end portion having a taper 132 configured to dilate tissue surrounding a puncture established with the needle 110. A proximal-end portion of the dilator 108 is disposed in the needle hub 106. Indeed, a proximal end of the dilator 108 is coupled to the distal wall 122 of the housing 116 of the needle hub 106. The dilator 108 is disposed over the needle 110 with at least the tip 134 of the needle 110 extending beyond a distal end of the dilator 108.

The needle 110 includes a distal-end portion having a tip 134 (e.g., a beveled tip) configured to establish a percutaneous puncture by inserting the tip 134 of the needle 110 into a blood vessel of a patient. A proximal-end portion of the needle 110 is disposed in the needle hub 106. Indeed, a proximal end of the needle 110 is coupled to the distal wall 122 of the housing 116 of the needle hub 106. The dilator 108 is disposed over the access guidewire 112. Due to the presence of the dilator 108, the needle 110 can be smaller than existing dialysis needles. This, in turn, can reduce anxiety in patients and novice clinicians alike.

In some embodiments, the needle 110 is retractable. In such embodiments, the needle 110 passes through the through hole 130 in the distal wall 122 and into the cavity 118 where the needle 110 is disposed within and coupled to a compressed compression spring. When actuated, a needle-retraction actuator releases the compressed compression spring allowing the compression spring to extend, thereby withdrawing the needle 110 into the needle hub 106.

The access guidewire 112 is configured to facilitate first-stick success upon establishing a percutaneous puncture with the needle 110 by ensuring novice clinicians that they are properly advancing the needle 110 into the correct location. The access guidewire 112 is disposed in the needle 110 in a mated or ready-to-deploy state of the cannulator 100. Indeed, the access guidewire 112 extends into the needle 110 from at least the cavity 118 by way of the through hole 130 in the distal wall 122 of the housing 116 of the needle hub 106. For example, when the guidewire actuator 114 is present in the compound needle 104, a proximal-end portion of the access guidewire 112 is coupled to the slider 136 of the guidewire actuator 114 and a remainder of the access guidewire 112 extends into the needle 110 from the cavity 118 by way of the through hole 130 in the distal wall 122 of the housing 116 of the needle hub 106. If the guidewire actuator 114 is not present, the access guidewire 112 additionally extends into the cavity 118 by way of the through hole in the proximal wall 120 of the housing 116 of the needle hub 106.

In some embodiments, first-stick success is further facilitated by visual confirmation of blood flashback such as through a translucent portion of the housing 116 or a window in the housing 116 fluidly connected by a channel to a lumen of the needle 110.

When present, the guidewire actuator 114 is configured to advance a distal end of the access guidewire 112 beyond the tip 134 of the needle 110 or withdraw the distal end of the access guidewire 112 into a distal-end portion the needle 110 proximal of the tip 134 of the needle 110. The guidewire actuator 114 can include a slider 136 slidably disposed in the longitudinal slot 128 of the housing 116 of the needle hub 106. The slider 136 includes an extension 138 coupled to the proximal-end portion of the access guidewire 112 in the body cavity 118 of the needle hub 106.

The compound cannula 102 includes a cannula 140 and a cannula hub 142 around a proximal-end portion of the cannula 140.

The cannula 140 includes a distal-end portion having a taper 144. Like the taper 132 of the dilator 108, the taper 144 of the cannula 140 is configured to dilate tissue surrounding a puncture established with the needle 110. Indeed, the taper 144 of the cannula 140 is configured to continue from the taper 132 of the dilator 108 in the mated state of the cannulator 100. The cannula 140 is formed of a flexible material such as a flexible polymeric material configured to increase patient comfort during dialysis. Indeed, the cannula 140 is in contrast to existing dialysis needles that are rigid (e g., metal) with a sharp tip that is left in place during dialysis. The sharp tip of such dialysis needles can result in infiltration (e.g., puncture of a blood-vessel wall) when the sharp tip is incorrectly advanced or a patient moves during dialysis, the latter of which is likely due to the duration of a dialysis treatment procedure.

The cannula hub 142 can be furcated (e.g., bifurcated). When furcated, the cannula hub 142 includes at least a primary arm 146 and a secondary arm 148 or side arm. The primary arm 146 of the cannula hub 142 includes a primary-arm lumen 150 and the secondary arm 148 of the cannula hub 142 includes a secondary-arm lumen 152. Each lumen of the primary-arm lumen 150 and the secondary-arm lumen 152 is fluidly connected to a cannula lumen 154 of the cannula 140. As shown, each arm of the primary arm 146 and the secondary arm 148 of the cannula hub 142 terminates in a connector such as a Luer connector, which can include a valve to seal the lumen after the compound needle 104 is removed from the compound cannula 102 in accordance with the needle-withdrawing step set forth below. This allows a dialysis treatment procedure to be performed without contaminating the immediate area with blood or other bodily fluids, thereby obviating health risks associated therewith. In addition, the valve keeps air from entering the compound cannula 102 when the connector is not connected to a closed system such as when dialysis components are disconnected or withdrawn from compound cannula 102.

The cannulator 100 has a mated or ready-to-deploy state of the compound needle 104 and the compound cannula 102 for at least cannulation with the cannulator 100. The mated state of the cannulator 100 includes the dilator 108 of the compound needle 104 disposed in the cannula 140 of the compound cannula 102 with a proximal-end portion of the cannula hub 142 coupled to the distal-end portion of the needle hub 106. Specifically, the proximal-end portion of the cannula hub 142 coupled to the distal-end portion of the needle hub 106 in the mated state of the cannulator 100 is the connector of the primary arm 146 of the cannula hub 142 disposed in the bore 126 in the distal-end portion of the needle hub 106.

### Methods

A method of the cannulator 100 includes cannulating a patient with the cannulator 100. Such a method includes a cannulator-obtaining step, a puncture-establishing step, an access guidewire-advancing step, a tissue-dilating step, a cannula-advancing step, and a needle-withdrawing step.

The cannulator-obtaining step includes obtaining the cannulator 100. As set forth above, the cannulator 100 includes the compound needle 104 and the compound cannula 102, preferably in the mated state of the cannulator 100.

The method can further include an ensuring step. The ensuring step includes ensuring the compound needle 104 and the compound hub are properly mated with the connector of the primary arm 146 of the cannula hub 142 disposed in the bore 126 of the distal- end portion of the needle hub 106. The ensuring step can also include ensuring the taper 132 of the distal-end portion of the dilator 108 continues along the taper 144 of the distal-end portion of the cannula 140. The ensuring step can also include ensuring the distal end of the guidewire 112 is not extended past the tip 134 of the needle 110 of the compound needle 104. When performed, the ensuring step should be performed prior to the puncture-establishing step.

The puncture-establishing step includes establishing a percutaneous puncture by inserting the tip 134 of the needle 110 of the compound needle 104 into a blood vessel (e.g., arteriovenous fistula) of the patient.

The access guidewire-advancing step includes advancing the distal end of the access guidewire 112 beyond the tip 134 of the needle 110 and into a blood-vessel lumen with the guidewire actuator 114 of the compound needle 104.

The access guidewire-advancing step includes distally sliding the slider 136 disposed in the longitudinal slot 128 of the needle hub 106. The slider 136 includes the extension 138 coupled to the proximal-end portion of the access guidewire 112 in the cavity 118 of the needle hub 106.

The tissue-dilating step includes dilating tissue surrounding the puncture with the taper 132 of the dilator 108 disposed over the needle 110. The tissue-dilating step also includes dilating the tissue with the taper 144 of the cannula 140.

The cannula-advancing step includes advancing the cannula 140 of the compound cannula 102 over the dilator 108, the needle 110, and the access guidewire 112 into the blood-vessel lumen.

The method can further include an access guidewire-withdrawing step. The access guidewire-withdrawing step includes withdrawing the distal end of the access guidewire 112 into the distal-end portion of the needle 110 proximal of the tip 134 of the needle 110 using the guidewire actuator 114, preferably before the needle-withdrawing step.

The needle-withdrawing step includes disconnecting the connector of the primary arm 146 of the cannula hub 142 from the bore 126 of the distal-end portion of the needle hub 106 and withdrawing the compound needle 104 from the blood-vessel lumen leaving the compound cannula 102 in place.

The method can further include a dialysis machine-connecting step and a dialysis-commencing step. The dialysis machine-connecting step includes connecting the compound cannula 102 to a tubing set of a dialysis machine. The dialysis-commencing step includes commencing dialysis through the compound cannula 102.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A cannulator (100) for cannulating an arteriovenous fistula, comprising:
a compound needle (104) including:
a needle (110);
a dilator (108) disposed over the needle (110) with at least a tip of the needle extending beyond a distal end of the dilator (108); and
a needle hub (106) around a proximal-end portion of the dilator (108); and
a compound cannula (102) including:
a cannula (140); and
a cannula hub (142) around a proximal-end portion of the cannula (140),
the cannulator (100) being capable of being arranged in a mated state of the compound needle (104) and the compound cannula (102) for at least cannulation with the cannulator (100), the mated state including the dilator (108) disposed in the cannula (140) with a proximal-end portion of the cannula hub (142) coupled to a distal-end portion of the needle hub (106), wherein the needle hub (106) includes a housing (116) enclosing a cavity (118) having a distal wall (122), each of a proximal end of the needle (110) and a proximal end of the dilator (108) coupled to the distal wall (122).

2. The cannulator of claim 1, the compound needle (104) further comprising an access guidewire (112) disposed in the needle (110), the access guidewire (112) extending from the cavity (118) through a through hole in the distal wall (122) of the needle hub (106).

3. The cannulator of claim 2, the compound needle further comprising a guidewire actuator (114) configured to advance a distal end of the access guidewire (112) beyond the tip of the needle (110) or withdraw the distal end of the guidewire into a distal-end portion of the needle (110) proximal of the tip of the needle (110).

4. The cannulator of claim 3, the guidewire actuator including a slider (136) slidably disposed in a longitudinal slot (128) of the needle hub (106), the slider (136) including an extension (138) coupled to a proximal-end portion of the access guidewire (112) in the cavity of the needle hub (106).

5. The cannulator of any claim of claims 1-4, wherein the cannula (140) is formed of a flexible polymeric material configured to increase patient comfort during a dialysis treatment procedure.

6. The cannulator of any claim of claims 1-5, wherein a distal-end portion of the cannula (140) includes a taper (144) configured to continue from a taper of the dilator (108) in the mated state of the cannulator.

7. The cannulator of any claim of claims 1-6, wherein the cannula hub (142) is furcated, the cannula hub including at least a primary arm (146) and a secondary arm (148).

8. The cannulator of claim 7, wherein the primary arm (146) of the cannula hub (142) includes a primary-arm lumen (150), the secondary arm (148) of the cannula hub includes a secondary-arm lumen (152), and each lumen of the primary-arm lumen (150) and the secondary-arm lumen (152) is fluidly connected to a cannula lumen (154) of the cannula.

9. The cannulator of either claim 7 or 8, wherein each arm of the primary arm (146) and the secondary arm (148) of the cannula hub (142) terminates in a Luer connector.

10. The cannulator of claim 9, wherein the proximal-end portion of the cannula hub (142) coupled to the distal-end portion of the needle hub (106) in the mated state of the cannulator is the Luer connector of the primary arm (146) of the cannula hub (142) disposed in a bore in the distal-end portion of the needle hub.

11. The cannulator according to one of the preceding claims, , the cannula having a Luer connector in a proximal-end portion of the cannula hub (142) that is arranged for being disposed in a bore of a distal-end portion of the needle hub (106).

12. The cannulator of claim 11, wherein a distal-end portion of the cannula (140) includes a taper configured to continue from a taper of the dilator (108) in the mated state of the cannulator.

## Patentansprüche

1. Kanülierungsvorrichtung (100) zum Kanülieren einer arteriovenösen Fistel, umfassend:
eine zusammengesetzte Nadel (104), einschließlich:
einer Nadel (110);
eines Dilatators (108), der über die Nadel (110) angeordnet ist, wobei sich zumindest eine Spitze der Nadel über ein distales Ende des Dilatators (108) hinaus erstreckt; und
eines Nadelansatzes (106) um einen proximalen Endabschnitt des Dilatators (108) herum; und
eine zusammengesetzte Kanüle (102), einschließlich:
einer Kanüle (140); und
eines Kanülenansatzes (142) um einen proximalen Endabschnitt der Kanüle (140) herum,
wobei die Kanülierungsvorrichtung (100) in einem verbundenen Zustand der zusammengesetzten Nadel (104) und der zusammengesetzten Kanüle (102) für zumindest eine Kanülierung mit der Kanülierungsvorrichtung (100) angeordnet sein kann, wobei der verbundene Zustand einschließt, dass der Dilatator (108) in der Kanüle (140) angeordnet ist, wobei ein proximaler Endabschnitt des Kanülenansatzes (142) mit einem distalen Endabschnitt des Nadelansatzes (106) verbunden ist, wobei der Nadelansatz (106) ein Gehäuse (116) einschließt, das einen Hohlraum (118) mit einer distalen Wand (122) umschließt, wobei jedes von einem proximalen Ende der Nadel (110) und einem proximalen Ende des Dilatators (108) mit der distalen Wand (122) verbunden ist.

2. Kanülierungsvorrichtung nach Anspruch 1, wobei die zusammengesetzte Nadel (104) weiter einen Zugangsführungsdraht (112) umfasst, der in der Nadel (110) angeordnet ist, wobei sich der Zugangsführungsdraht (112) von dem Hohlraum (118) durch eine Durchgangsbohrung in der distalen Wand (122) des Nadelansatzes (106) erstreckt.

3. Kanülierungsvorrichtung nach Anspruch 2, wobei die zusammengesetzte Nadel weiter ein Führungsdraht-Betätigungselement (114) umfasst, das zum Vorschieben eines distalen Endes des Zugangsführungsdrahts (112) über die Spitze der Nadel (110) hinaus oder zum Zurückziehen des distalen Endes des Führungsdrahts in einen distalen Endabschnitt der Nadel (110), der proximal zu der Spitze der Nadel (110) liegt, konfiguriert ist.

4. Kanülierungsvorrichtung nach Anspruch 3, wobei das Führungsdraht-Betätigungselement ein Schiebeelement (136) einschließt, das verschiebbar in einem Längsschlitz (128) des Nadelansatzes (106) angeordnet ist, wobei das Schiebeelement (136) eine Erweiterung (138) einschließt, die mit einem proximalen Endabschnitt des Zugangsführungsdrahts (112) in dem Hohlraum des Nadelansatzes (106) verbunden ist.

5. Kanülierungsvorrichtung nach einem der Ansprüche 1-4, wobei die Kanüle (140) aus einem flexiblen Polymermaterial gebildet ist, das zum Erhöhen des Patientenkomforts während eines Dialyse-Behandlungsvorgangs konfiguriert ist.

6. Kanülierungsvorrichtung nach einem der Ansprüche 1-5, wobei ein distaler Endabschnitt der Kanüle (140) eine Verjüngung (144) einschließt, die zum Fortsetzen von einer Verjüngung des Dilatators (108) in dem verbundenen Zustand der Kanülierungsvorrichtung konfiguriert ist.

7. Kanülierungsvorrichtung nach einem der Ansprüche 1-6, wobei der Kanülenansatz (142) gegabelt ist und der Kanülenansatz zumindest einen primären Schenkel (146) und einen sekundären Schenkel (148) einschließt.

8. Kanülierungsvorrichtung nach Anspruch 7, wobei der primäre Schenkel (146) des Kanülenansatzes (142) ein primäres Schenkellumen (150) einschließt, der sekundäre Schenkel (148) des Kanülenansatzes ein sekundäres Schenkellumen (152) einschließt, und jedes Lumen des primären Schenkellumens (150) und des sekundären Schenkellumens (152) in Fluidverbindung mit einem Kanülenlumen (154) der Kanüle steht.

9. Kanülierungsvorrichtung nach Anspruch 7 oder 8, wobei jeder Schenkel des primären Schenkels (146) und des sekundären Schenkels (148) des Kanülenansatzes (142) in einem Luer-Verbinder endet.

10. Kanülierungsvorrichtung nach Anspruch 9, wobei es sich bei dem proximalen Endabschnitt des Kanülenansatzes (142), der mit dem distalen Endabschnitt des Nadelansatzes (106) in dem verbundenen Zustand der Kanülierungsvorrichtung verbunden ist, um den Luer-Verbinder des primären Schenkels (146) des Kanülenansatzes (142) handelt, der in einer Bohrung in dem distalen Endabschnitt des Nadelansatzes angeordnet ist.

11. Kanülierungsvorrichtung nach einem der vorstehenden Ansprüche, wobei die Kanüle einen Luer-Verbinder in einem proximalen Endabschnitt des Kanülenansatzes (142) aufweist, der dafür angeordnet ist, in einer Bohrung eines distalen Endabschnitts des Nadelansatzes (106) angeordnet zu sein.

12. Kanülierungsvorrichtung nach Anspruch 11, wobei ein distaler Endabschnitt der Kanüle (140) eine Verjüngung einschließt, die zum Fortsetzen von einer Verjüngung des Dilatators (108) in dem verbundenen Zustand der Kanülierungsvorrichtung konfiguriert ist.

## Revendications

1. Canulateur (100) pour canuler une fistule artérioveineuse, comprenant :
un composant aiguille (104) incluant :
une aiguille (110) ;
un dilatateur (108) disposé sur l'aiguille (110) avec au moins une pointe de l'aiguille s'étendant au-delà d'une extrémité distale du dilatateur (108) ; et
un moyeu d'aiguille (106) autour d'une partie d'extrémité proximale du dilatateur (108) ; et
un composant canule (102) incluant :
une canule (140) ; et
un moyeu de canule (142) autour d'une partie d'extrémité proximale de la canule (140),
le canulateur (100) pouvant être agencé dans un état accouplé du composant aiguille (104) et du composant canule (102) pour au moins une canulation avec le canulateur (100), l'état accouplé incluant le dilatateur (108) disposé dans la canule (140) avec une partie d'extrémité proximale du moyeu de canule (142) couplée à une partie d'extrémité distale du moyeu d'aiguille (106), dans lequel le moyeu d'aiguille (106) inclut un boîtier (116) renfermant une cavité (118) présentant une paroi distale (122), chacune d'une extrémité proximale de l'aiguille (110) et d'une extrémité proximale du dilatateur (108) étant couplée à la paroi distale (122).

2. Canulateur selon la revendication 1, le composant aiguille (104) comprenant en outre un fil-guide d'accès (112) disposé dans l'aiguille (110), le fil-guide d'accès (112) s'étendant à partir de la cavité (118) à travers un trou traversant dans la paroi distale (122) du moyeu d'aiguille (106).

3. Canulateur selon la revendication 2, le composant aiguille comprenant en outre un actionneur de fil-guide (114) configuré pour faire avancer une extrémité distale du fil-guide d'accès (112) au-delà de la pointe de l'aiguille (110) ou retirer l'extrémité distale du fil-guide dans une partie d'extrémité distale de l'aiguille (110) proximale de la pointe de l'aiguille (110).

4. Canulateur selon la revendication 3, l'actionneur de fil-guide incluant un coulisseau (136) disposé de manière coulissante dans une fente longitudinale (128) du moyeu d'aiguille (106), le coulisseau (136) incluant une extension (138) couplée à une partie d'extrémité proximale du fil-guide d'accès (112) dans la cavité du moyeu d'aiguille (106).

5. Canulateur selon l'une quelconque des revendications 1 à 4, dans lequel la canule (140) est formée d'un matériau polymère flexible configuré pour augmenter le confort du patient pendant une procédure de traitement par dialyse.

6. Canulateur selon l'une quelconque des revendications 1 à 5, dans lequel une partie d'extrémité distale de la canule (140) inclut un cône (144) configuré pour continuer à partir d'un cône du dilatateur (108) dans l'état accouplé du canulateur.

7. Canulateur selon l'une quelconque des revendications 1 à 6, dans lequel le moyeu de canule (142) est ramifié, le moyeu de canule incluant au moins un bras primaire (146) et un bras secondaire (148).

8. Canulateur selon la revendication 7, dans lequel le bras primaire (146) du moyeu de canule (142) inclut une lumière de bras primaire (150), le bras secondaire (148) du moyeu de canule inclut une lumière de bras secondaire (152), et chaque lumière de la lumière de bras primaire (150) et de la lumière de bras secondaire (152) est fluidiquement raccordée à une lumière de canule (154) de la canule.

9. Canulateur selon l'une ou l'autre des revendications 7 et 8, dans lequel chaque bras du bras primaire (146) et du bras secondaire (148) du moyeu de canule (142) se termine par un raccord Luer.

10. Canulateur selon la revendication 9, dans lequel la partie d'extrémité proximale du moyeu de canule (142) couplée à la partie d'extrémité distale du moyeu d'aiguille (106) dans l'état accouplé du canulateur est le raccord Luer du bras primaire (146) du moyeu de canule (142) disposé dans un alésage dans la partie d'extrémité distale du moyeu d'aiguille.

11. Canulateur selon l'une des revendications précédentes, la canule présentant un raccord Luer dans une partie d'extrémité proximale du moyeu de canule (142) qui est agencée pour être disposée dans un alésage d'une partie d'extrémité distale du moyeu d'aiguille (106).

12. Canulateur selon la revendication 11, dans lequel une partie d'extrémité distale de la canule (140) inclut un cône configuré pour continuer à partir du cône du dilatateur (108) dans l'état accouplé du canulateur.
